# EUROPEAN PATENT SPECIFICATION

(11) **EP 2 883 531 B1**
(45) Date of publication and mention of the grant of the patent: **15.06.2022**
(21) Application number: 13197052.7
(22) Date of filing: 13.12.2013
(51) Int. Cl.: A61K 8/34, A61Q 5/08, A61K 8/49, A61Q 5/06

(54) **Bleaching and coloring composition for hair**
Bleich- und Färbezusammensetzung für Haare
Composition de blanchiment et de coloration pour les cheveux

(43) Date of publication of application: 17.06.2015
(73) Proprietor: Kao Corporation, Chuo-ku Tokyo 103-8210 (JP)
(72) Inventor: Ächtner, Anja, 64297 Darmstadt (DE); Pratt, Dominic, 64297 Darmstadt (DE); Hänsel, Clarissa, 64297 Darmstadt (DE); Möhring, Hartmut, 64297 Darmstadt (DE)
(74) Representative: Hoffmann Eitle

(56) References cited:
- WO-A2-2009/005139
- WO-A2-2013/092904

## Description

Present invention relates to an aqueous composition for bleaching and coloring hair which comprises a specific anionic direct dye and an oxidizable solvent in addition to a compound with a bleaching effect, an alkalizing agent and an oxidizing agent.

Bleaching and dyeing hair are always done in two subsequent process as the most of the direct dyes if not all are not stable under highly oxidative conditions.

In order to achieve darker and vivid colours this is essential. Exposure of hair to two different process one after another furthermore causes considerable hair damage.

The purpose of the present invention is to find out a new process wherein hair is bleached and coloured in the same process. The purpose is at the same time finding out a new composition wherein the direct dyes have considerably improved stability so that it allows achieving intensive and vivid colors on hair.

The inventors of the present invention have found out that that a composition co nprising a direct dye of a particular chemical structure, at least one compound with bleaching effect, at least one oxidizing agent, at least one alkalizing agent and at least one oxidizable solvent bleaches and colours hair intensively due to the improved stability of the particular direct dye otherwise instable in the same system. At the same time the composition colours hair homogeneously especially hair with parts having various degree of damage in its length. It is also observed that the composition is milder to the hair than the same composition without oxidizable solvent.

WO 2009/005139A2 describes a hair dye composition comprising a specific azo dye of a certain formula. The hair dye composition may be an oxidative dyeing or bleaching composition further comprising an oxidizing agent.

EP 2 606 875 A1 (corresponding to WO 2013/092904A2) discloses bleaching and coloring compositions comprising at least one direct dye. However, the document does not disclose directly and unambiguously the direct dye of the present invention and also fails to teach compositions comprising highly stable direct dyes.

Thus, the first object of the present invention is an aqueous composition for bleaching and coloring hair comprising at least one direct dye of the chemical structure 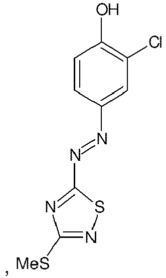 at least one compound with hair bleaching effect, at least one oxidizing agent, at least one alkalizing agent and at least one oxidizable solvent selected from 2-phenoxyethanol and benzyl alcohol.

The second object of the present invention is the use of the composition for bleaching and coloring hair, especially human hair.

It is known in the art that bleaching compositions are prepared by mixing at least two compositions prior to application onto hair. Therefore, present invention is at the same time on a three part bleaching coloring composition for hair wherein the first part, Part A, is an anhydrous composition and comprises direct dye of the above chemical structure, at least one compound with hair bleaching effect and at least one alkalizing agent, the second part, Part B, is an aqueous composition and comprises at least one oxidizing agent and the third part, Part C, comprises at least one oxidizable solvent.

Further object of the present invention is a method for bleaching and coloring hair wherein an aqueous composition is prepared by mixing immediately before use an anhydrous composition (Part A) comprising direct dye of the above chemical structure, at least one compound with hair bleaching effect and at least one alkalizing agent, and an aqueous composition (Part B) comprising at least one oxidizing agent and a composition (Part C) comprising at least one oxidizable solvent and applied onto hair and after processing 5 to 45 min rinsed off from hair.

Further object of the present invention is a kit for bleaching and colouring hair comprising three of more compositions wherein it comprises a Part A which is an anhydrous composition and comprises direct dye of the above chemical structure, at least one compound with bleaching effect and at least one alkalizing agent, and a Part B which is an aqueous composition comprising at least one oxidizing agent and a Part C comprising at least one oxidizable solvent.

With the term oxidizable solvent it is meant that the compound is oxidized under the conditions used to bleach and color the hair.

It is furthermore the object of the present invention is the use of oxidizable solvent, preferably an aromatic or aliphatic alcohol for stabilizing the direct dye of the above chemical structure in an aqueous composition comprising at least one compound with hair bleaching effect, at least one oxidizing agent and at least one alkalizing agent.

With the term anhydrous, it is meant that no additional water or any aqueous composition is added to the composition. However, it should be understood that the raw materials used may comprise water at low concentrations and therefore, from the experience, up to 1% water content does not have any influence on the stability and incorporated into compositions of the present invention in the form of bound water of individual chemicals.

Composition of the present invention comprises the direct dye of the above chemical structure at a concentration of 0.01 to 10%, preferably 0.05 to 7.5% and more preferably 0.2 to 5% by weight calculated to the total composition.

It should be noted that throughout the description all concentrations are given as % by weight calculated to the total composition in the respective composition prior to mixing with any other parts.

The anhydrous composition may comprise additional direct dyes known in the art such as anionic, cationic and nonionic ones. Plant dyes are also suitable for the compositions of the present invention.

Suitable anionic direct dyes in aqueous composition are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No.8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No.8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. Among those, the most preferred anionic dyestuffs are Acid Red 52, DC Violet 2, DC Red 33, DC Orange 4, DC Red 27 and DC Yellow 10.

Suitable cationic dyes in aqueous composition are in principal those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87 and Basic Orange 31. The most preferred ones are Basic red 51, Basic Yellow 87 and Basic Orange 31 sold by CIBA.

Additionally, the aqueous compositions of the present invention may comprise neutral dyes (HC dyes), so called nitro dyes for shading purposes. Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic directacting dyestuffs, for example henna (red or black), alkanna root, laccaic acid, indigo, logwood powder, madder root and rhubarb powder, etc.

Further suitable direct dyes which are anionic under alkaline conditions are according to the following structures: 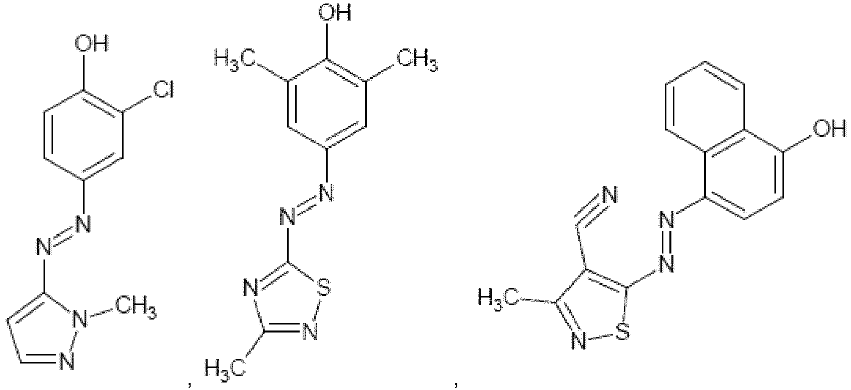 and

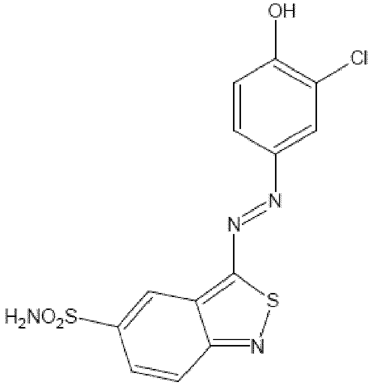

From the above disclosed direct dyes the preferred are anionic and nitro dyes and most preferred are anionic direct dyes.

According to the invention, the composition comprises one or more direct dye at a concentration of 0.01 to 10%, preferably 0.05 to 7.5% and more preferably 0.2 to 5% by weight calculated to the total composition. The composition can also comprise mixture of several direct dyes i.e. an anionic, a cationic and/or a nonionic ones. In such a case the dyes may be mixed at any ratio with each other.

The above mentioned direct dyes of cationic, anionic and nonionic character are preferably comprised in the water free bleaching composition at the concentration given in the above paragraph. The direct dyes of different characters can certainly be mixed as well.

Compositions of the present invention comprise at least one compound with hair bleaching effect. Suitable compounds are in general peroxides. Useful as such are in particular persulfates such as sodium and potassium persulfate, ammonium persulfate, earth alkali peroxides such as magnesium peroxide, melamine peroxide or urea peroxide or phtholimidoperoxy hexanoic acid. The proportion of peroxides is at least 5%, preferably in the range of 10 to 80%, more preferably 15 to 60% and most preferably 20 to 50% by weight, calculated to total composition. The compounds with hair bleaching effect are comprised preferably in anhydrous composition (Part A)

According to the invention, the anhydrous composition can also comprise 0.1% to 10% by weight, calculated to total composition prior to mixing with oxidizing lotion, at least one ammonium salts. Suitable ammonium salts are ammonium carbonate, ammonium hydrogen carbonate, ammonium carbamate, ammonium chloride, ammonium sulfate, ammonium phosphates, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium tartarate, ammonium benzoate, ammonium acetate, ammonium formiate and ammonium lactate. Compositions may also comprise mixture or ammonium salts.

Preferred thereof are the ammonium phosphates, such as ammonium dihydrogen phosphate, diammonium hydrogen phosphate, diammonium sodium phosphate, sodium ammonium hydrogen phosphate or ammonium disodium phosphate, ammonium chloride, ammonium sulfate and diammonium hydrogen citrate, as well as ammonium chloride.

As known from EP 609 796 A2, the ammonium compounds can also be used as sole bleaching agent in respectively higher amounts.

The total proportion of the compounds with bleaching and/or highlighting effect preferably ranges from 5% to 85%, preferably 10% to 80%, more preferably 15 to 70% and most preferably 20 to 60% by weight calculated to total composition.

Anhydrous composition comprises at least one alkalizing agent. Preferred alkalizing agent is sodium metasilicate and preferably comprised at a concentration 1 to 20% by weight calculated to the total composition. It should be noted that the composition applied onto hair is an alkaline composition and main alkalizing agent is in the anhydrous composition and the concentration must be enough to achieve a pH between 8 and 12. The pH of the ready to use product, mixture of bleaching composition, oxidizing lotion and oxidizable solvent, is preferably in the range of 8 to 11.5, in particular between 9 and 11.

In addition to the active component, anhydrous compositions also comprise the components customarily used in such compositions: In particular inert pulverulent carrier materials, these are for example, pyrogenic silicium dioxide, starch powder, etc., surface-active substances, binding agents, etc.. In order to avoid repetition, reference is made to the respective standard literature, for example, K. Schrader and A. Domsch, "Cosmetology - Theory and Practice (2005, Verlag für Chemische Industrie), pages 142 to 151.

The ready to use composition is prepared by mixing the components with a composition comprising at least one oxidizing agent (Part B). The oxidizing agents suitable are hydrogen peroxide, urea peroxide, melamin peroxide or perborate salts. The most preferred is hydrogen peroxide. Such composition comprises 2 to 12% by weight at least one oxidizing agent preferably hydrogen peroxide and is either a solution or in the form of an emulsion.

The compositions of the present invention comprises at least one oxidizable solvent. With the term oxidizable it is meant that the solvent is oxidized, at least partly, which may also be as a whole under the conditions used for bleaching and colouring hair.

According to the present invention, the oxidizable solvent is selected from 2-phenoxyethanol and benzyl alcohol.

At least one oxidizable solvent is comprised in the composition of the present invention is at a concentration below 8%, preferably 0.1 to 7.5% and more preferably 0.2 to 6% and most preferably 0.25 to 5% by weight calculate to the total composition.

Compositions of the present invention may comprise one or more of the ingredients disclosed in the following. They may be comprised in one or more parts of Part A, B and C. In the selection of particular composition compatibility of the selected ingredient in the particular part of the composition must be taken into account in case the compositions are prepared by mixing the parts after a long storage period.

Composition of the present invention may further comprise lipophilic ingredients such as vegetable oils, for example, jojoba oil or any other; petrolatum liquid paraffins, especially paraffinum perliquidum and parafiinum subliquidum; silicone oils; hydropobic fatty acid esters such as octyl palmitate, isocetyl palmitate, isopropyl palmitate and octyl stearate, C₁₀- to C₃₆-fatty acid triglycerides, as well as their mixtures. In the case that the use is wished among those the most preferred ones are silicone oils, jojoba oil, fatty acid esters, paraffin oils, combinations of fatty acid esters and paraffin oils. Fatty acid esters and/or paraffin oils and/or silicone oils are particularly preferred. Concentration of these lipophilic compounds are used in a total amount of about 0.1 to 20 percent by weight, preferably from 1 to 15 percent by weight, and more preferably from 2 to 10 percent by weight, calculated to total composition prior to mixing with oxidizing lotion.

In principal any silicone oil is useful as a lipophilic compound. Preferred are arylated silicones as a lipophilic ingredient at a concentration range of 0.1 to 50%, preferably 0.5 to 40% more preferably 1 to 35% and most preferably 2.5 to 30% by weight calculated to total composition prior to mixing with oxidizing lotion. Non-limiting suitable examples are phenyl methicone, phenyl trimethicone, diphenyl dimethicone, diphenylsiloxy phenyl trimethicone, tetramethyl tetraphenyl trisiloxane, triphenyl trimethicone, tetramethly tetraphenyl trisiloxane and pentaphenyl trimethyl trisiloxane.

In the preferred embodiment of the present invention, the arylated silicone comprises at least 2 phenyl groups, more preferably 3 and most preferably 5 phenyl groups in its molecule.

Particularly preferred arylated silicone is pentaphenyl trimethyl trisiloxane available from Dow Corning under the trade name DC PH-1555 HRI.

It should be noted that compositions of the present invention can also comprise more than one arylated silicone.

Further, compositions may comprise polymers selected from the group consisting of cellulose polymer compounds, alginate, polysaccarides and acrylic acid polymers, preferably methyl cellulose compounds, ethyl cellulose compounds, hydroxyethylcellulose compounds, methylhydroxyethylcellulose compounds, methylhydroxypropylcellulose compounds, carboxymethyl cellulose compounds, alginic acids, sodium alginates, ammonium alginates, calcium alginates, gum arabic, guar gum or xanthan gum, or acrylic acid polymers with molecular weights from about 1,250,000 to 4,000,000, alone or in combination with each other. The polymers are used in a total concentration of 0.1 to 15 %, preferably from 0.2 to 10 %, and more preferably in an amount of from 0.5 to 7.5% by weight, calculated to total composition.

Composition can also comprise cationic polymers as conditioning and/or thickening agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 4, Polyquaternium 6, Polyquaternium 7, Polyquaternium 10, Polyquaternium 11, Polyquaternium 16, Polyquaternium 22, Polyquaternium 24, Polyquaternium 28, Polyquaternium 30, Polyquaternium 37, Polyquaternium 36, Polyquaternium 46, Polyquaternium 67, and Polyquaternium 72.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

Typical concentration range for any of the cationic conditioners mentioned above can be 0.1 - 7.5% by weight, preferably 0.3 - 5% by weight and more preferably 0.5 - 2.5% by weight, calculated to total composition

Especially the composition comprising at least one oxidizing agent (Part B) may be in the form of emulsion, solution, dispersion and/or gel. Emulsion is the preferred form.

In the case the composition is in the form of an emulsion, it comprises as an emulsion base at least one fatty alcohol or mixture of fatty alcohols with the chain length of 14 to 22 C atoms. Examples to suitable fatty alcohols, without limiting the choice, are myristyl alcohol, cetyl alcohol, stearyl alcohol, behenyl alcohol and cetostearyl alcohol. The most preferred is cetostearyl alcohol well known with its trade name Lanette O or as Lanette N in mixture with sodium cetearyl sulfate from Cognis.

The concentration of fatty alcohol(s) is in the range from 0.5 to 20%, preferably 1 to 15% by weight, calculated to total composition prior to mixing with oxidizing and bleaching and/or highlighting composition.

Compositions according to present invention may comprise surfactants selected from anionic, nonionic, amphoteric (or zwiterionic) and/or cationic surfactants as emulsifier or solubilizer. Cationic surfactants are as well used as hair conditioners in the compositions.

Anionic surfactants suitable within the scope of the invention are in principal known from the cleansing compositions

These are anionic surfactants of the sulfate, sulfonate, carboxylate and alkyl phosphate type, for example, the known C₁₀-C₁₈-alkyl sulfates, and in particular the respective ether sulfates, for example, C₁₂₋C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof as well as alkyl amido polyether carboxylic acids and salts thereof. Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants.

An overview of the anionic surfactants suitable for the present invention can furthermore be found in the monography of K. Schrader, "Grundlagen und Rezepturen der Kosmetika", 2nd Ed.(1989, Hüthig Buchverlag), pp. 595-600 and pp. 683 to 691.

Further surfactants in the compositions according to the invention are nonionic surfactants alone or in admixture with anionic surfactants. These are described as well in Schrader, I.c., on pages 600-601 and pp. 694-695. Especially suited nonionic surfactants are, for example, long-chain fatty acid mono- and dialkanolamides, such as coco fatty acid mono- or diethanolamide and myristic fatty acid mono or diethanolamide, stearic acid mono or diethanolamide. Further nonionic surfactants suited are alkyl polyglucosides with an alkyl group of 8 to 18 carbon atoms, and with 1 to 5 glucoside units. Further additionally useful nonionic surfactants are, for example, the various sorbitan esters, such as polyethylene glycol sorbitan stearic acid ester, fatty acid polyglycol esters or polycondensates of ethyleneoxide and propyleneoxide, as they are on the market, for example, under the trade name "Pluronics^{R}", as well as fatty alcohol ethoxylates. Further nonionic surfactants preferred in the dyeing compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 25, preferably about 10 and about 20.

As further surfactant component, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

Composition can contain cationic surfactants as emulsifier, solubilizer and/or conditioning ingredients according to the formula, but not limited to. 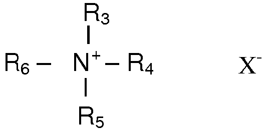 where R₃ is a saturated or unsaturated, branched or linear alkyl chain with 8-22 C atoms or

R₇ CO NH (CH₂)ₙ

where R₇ is saturated or unsaturated, branched or linear alkyl chain with 7-21 C atoms and n has typical value of 1 - 4 or

R₈ CO O (CH₂)ₙ

where R₈ is saturated or unsaturated, branched or linear alkyl chain with 7-21 C atoms and n has typical value of 1 - 4, and
R₄ is H or unsaturated or saturated, branched or linear alkyl chain with 1 - 22 C atoms or

R₇CONH(CH₂)ₙ

or

R₈ CO O (CH₂)ₙ

where R₇, R₈ and n are same as above.

R₅ and R₆ are H or lower alkyl chain with 1 to 4 Carbon atoms, and X is typically chloride, bromide, methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Concentration of one or more surfactants in dyeing composition is in the range of 0.1 to 20%, preferably 0.2 to 15% and most preferably 0.2 - 10% by weight, calculated to the total composition.

Compositions of the present invention can comprise an organopolysiloxane wherein at least one silicium atom is linked to an alkylene group having a heteroatom, in particular a nitrogen atom, with a poly-(N-acyl alkyleneimine) units of the formula 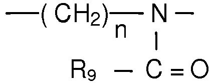 wherein n is a number from 1 to 5 and R₉ is hydrogen, a C₁-C₁₂-alkyl or cycloalkyl, aralkyl or aryl group. Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 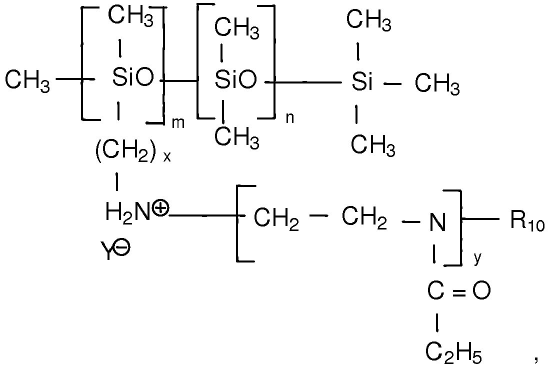 wherein m and n each are numbers from 20 to 10,000, in particular 50 to 7,000, especially 100 to 5,000, x is a number between 1 and 5, preferably 3, and y is a number from 5 to 30, R₁₀ is a C₁-C₁₂-alkyl or aryl group, in particular a methyl, ethyl or benzyl group, and Y⁻ is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05% to 5%, preferably 0.1% to 2.5%, in particular 0.5% to 1.5% by weight, calculated to the total composition.

Another preferred compound in the composition of present invention especially in bleaching and/or highlighting composition and in dyeing composition is ceramide type of compounds according to general formula 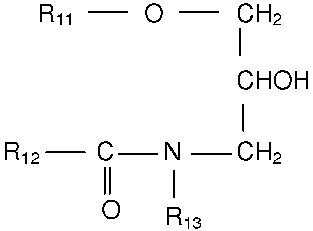 where R₁₁ and R₁₂ are independent from each other alkyl- or. alkenyl group with 10 to 22 carbon atoms and R₁₃ is alkyl or hydroxyl alkyl with 1 to 4 carbon atoms group. Preferred compound according to the above chemical structure is cetyl-PG-hydroxyethylpalmitamide. Concentration of ceramide type of compounds ranges from 0.01 to 2%, preferably 0.01 to 1% yb weight calculated to total composition before mixing.

The compositions of the present invention can comprise of at least one ubiquinone of the formula (I) 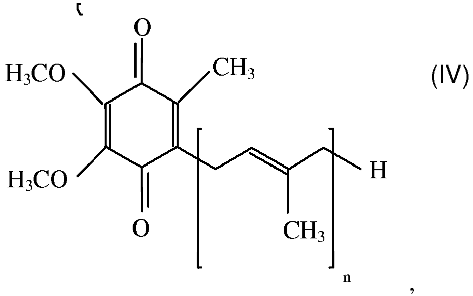 wherein n is a number from 1 to 10. Concentration of ubichinone can vary between 0.001 % and 10 % by weight, calculated to the total composition before mixing.

Compositions of the present invention may comprise synthetic mica coated with metal oxide or oxides having a volume particle size distribution in the range of 1 to 750 µm. Use of synthetic mica coated with metal oxide or oxides mainly in decorative cosmetics is disclosed in an international patent application of Sun Chemical Corporation published with a number WO 2005/065632 A1. In the document synthetic mica and coated synthetic mica with at least one metal oxide or oxides is disclosed in detail. The content of the document is included herewith by reference.

Suitable metal oxide or oxides for coating synthetic mica are titanium dioxide, chromium oxide, ferric oxide or mixtures thereof. In the present invention the preferred is synthetic mica coated with titanium dioxide. Such materials are commercially available from Sun Chemical Corporation and Merck (Timiron Synwhite 40) and known with their INCI names Synthetic Fluorphologopite

The volume particle size distribution of synthetic mica coated with a metal oxide or oxides is in the range of 1 to 750 µm, preferably 1 to 250 µm, more preferably 1 to 100 µm and most preferably 5 to 95 µm. The particle sizes referred are relating to the volume particle size distribution meaning that particles found in the coated synthetic mica having volume particle size in the given ranges.

Concentration of synthetic mica coated with at least metal oxide or oxides is from 0.01 to 20%, preferably 0.1 to 15%, more preferably 0.25 to 10% and most preferably 0.5 to 55% by weight calculated to total composition.

Compositions may further comprise additional substances found in lightening and/or coloring compositions for hair such as fragrance, humectants, chelants and radical scavengers.

The following examples are to illustrate the invention but not to limit it.

### Example 1

### Bleaching - colouring powder composition - Part A

| | |
|---|---|
| Hydroxyethylcellulose | 1.40% by weight |
| Cellulose gum | 3.20 |
| Xanthan gum | 0.30 |
| Tetrasodium EDTA | 2.00 |
| Sodium carbonate | 1.00 |
| Ammonium persulfate | 11.00 |
| Potassium persulfate | 36.00 |
| Sodium metasilicate | 10.00 |
| Corn starch | 1.10 |
| Diatomaceous Earth | q.s. to 100 |
| Polyquaternium - 10 | 0.10 |
| Silica* | 1.00 |
| Dyestuff** | 0.10 |

| | |
|---|---|
| **: the dyestuff of the chemical structure in claim 1 *: Aerosil 380 | |

The above composition is prepared by combining all powder components together and by mixing until homogeneity in a suitable mixer.

### Oxidizing Lotion - Part B

| | |
|---|---|
| Hydrogen peroxide | 9.00 (% by wt.) |
| Cetyl stearyl alcohol | 1.70 |
| Phosphoric acid | 0.50 |
| Sodium lauryl sulfate | 0.20 |
| Coenzyme Q10 | 0.05 |
| Cetyl PG hydroxyethyl palmitamide | 0.10 |
| Disodium hydrogen phosphate | 0.10 |
| Salicylic acid | 0.10 |
| Water | ad 100.00 |

### Oxidizable Solvent Composition - Part C

| | |
|---|---|
| Benzyl alcohol | 100 |

Form the above three compositions a ready to use composition was prepared as follows:

| | Inventive | Comparative |
|---|---|---|
| Part A | 5 g | 5g |
| Part B | 7g | 7g |
| Part C | 0.36 g | - |
| Water | - | 0.36 g |

Both compositions were allowed to stand for 30 min at room temperature. The dye concentration in both compositions was measured after dissolving the dyestuff by dispersing the whole mixed composition in isopropanol and subsequent filtration with a colorimeter It was observed that in the composition of the present invention almost no dyestuff degradation occurred whereas in the comparative composition the dyestuff was almost completely degraded.

At the same time, hair streaks were bleached and colored with the aqueous compositions as above prepared freshly and stored 30 min at ambient temperature at 40°C for 30 min. After rinsing and drying hair, L, a and b values were measured for each tress colored with freshly prepared composition and composition stored for 30 min at ambient temperature and using the values the colour differences (ΔE) were calculated between hair tresses colored with freshly prepared composition and colored with composition stored for 30 min at ambient temperature. The following results were obtained.

| | ΔE |
|---|---|
| Inventive | 0.45 |
| Comparative | 2.16 |

From the above it is clear that the dyestuff is stabile in the inventive composition which is evident that there is no visible color difference between the tresses colored with freshly prepared composition and colored with composition stored for 30 min at ambient temperature whereas the dyestuff is stabile in the comparative composition which is evident that there is a clear visible color difference between the tresses colored with freshly prepared composition and colored with composition stored for 30 min at ambient temperature.

Form the above results, it is concluded that presence of benzyl alcohol in the aqueous composition stabilizes the direct dye.

The following examples are within the scope of the present invention.

### Example 2

### Bleaching - colouring powder composition - Part A

| | |
|---|---|
| Hydroxyethylcellulose | 1.40% by weight |
| Cellulose gum | 3.20 |
| Xanthan gum | 0.30 |
| Tetrasodium EDTA | 2.00 |
| Sodium carbonate | 1.00 |
| Ammonium persulfate | 21.00 |
| Potassium persulfate | 46.60 |
| Sodium metasilicate | 10.20 |
| Corn starch | 1.10 |
| Diatomaceous Earth | q.s. to 100 |
| Polyquaternium - 10 | 0.10 |
| Silica* | 1.00 |
| Dyestuff-1** | 0.10 |
| Dyestuff-2*** | 0.10 |

| | |
|---|---|
| **: the dyestuff of the chemical structure in claim 1 *: Aerosil 380 ***: The dyestuff of the chemical structure | |

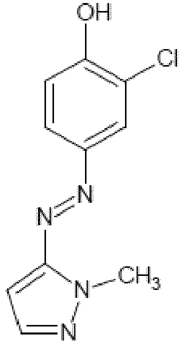

The bleaching - colouring compositions were prepared in the same way as in example 1 except as the composition C, pure was used. The coloring of the streaks was carried out in the same way as in Example 1. Similar results were observed as in the case of Example 1.

Similar results were observed when phenoxyethanol was replaced with each of benzyl alcohol and 2-benzyloxyethanol.

### Example 3

### Bleaching - colouring powder composition - Part A

| | |
|---|---|
| Hydroxyethylcellulose | 1.40% by weight |
| Cellulose gum | 3.20 |
| Xanthan gum | 0.30 |
| Tetrasodium EDTA | 2.00 |
| Sodium carbonate | 1.00 |
| Ammonium persulfate | 21.00 |
| Potassium persulfate | 46.60 |
| Sodium metasilicate | 10.20 |
| Corn starch | 1.10 |
| Diatomaceous Earth | q.s. to 100 |
| Polyquaternium - 10 | 0.10 |
| Silica* | 1.00 |
| Dyestuff -1 *** | 0.10 |
| Acid red 92 | 0.10 |

Similar results were observed as in example 1 when benzyl alcohol, phenoxyethanol and 2-benzyloxyethanol was used as composition C in combination with the oxidizing composition of the example 1

## Claims

1. An aqueous composition for bleaching and colouring hair **characterized in that** it comprises at least one compound with hair bleaching effect, at least one oxidizing agent different from the compound with hair bleaching effect, at least one alkalizing agent and at least one oxidizable solvent selected from 2-phenoxyethanol and benzyl alcohol and the dyestuff of the chemical structure

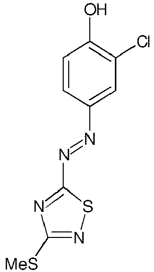

2. The composition according to claim 1 **characterized in that** at least one compound with bleaching effect is selected from persulphates such as sodium persulphate, potassium persulphate and ammonium persulphate or their mixtures, and preferably at a concentration of at least 5% by weight calculated to the total composition.

3. The composition according to claims 1 and 2 **characterized in that** it comprises additionally at least one direct dye selected from cationic, anionic and nonionic dyes.

4. The composition according to any of the preceding claims **characterized in that** at least one additional direct dye is an anionic direct dye and
preferably selected from Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No.8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No.8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. Among those, the most preferred anionic dyestuffs are Acid Red 52, DC Violet 2, DC Red 33, DC Orange 4, DC Red 27, DC Yellow 10.

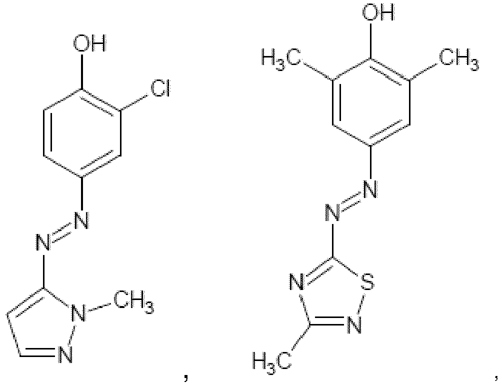

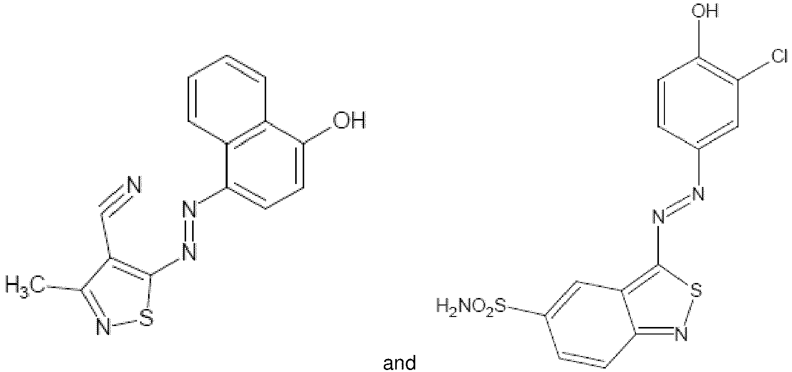

5. The composition according to any of the preceding claims **characterized in that** at least one oxidizing agent is hydrogen peroxide.

6. The composition according to any of the preceding claims **characterized in that** at least one alkalizing agent is sodium metasilicate.

7. The composition according to any of the preceding claims **characterized in that** at least one oxidizable solvent is comprised at a concentration below 8%, preferably 0.1 to 7.5% and more preferably 0.2 to 6% and most preferably 0.25 to 5% by weight calculate to the total composition.

8. The composition according to any of the preceding claims **characterized in that** it comprises at least one cationic polymer.

9. Use of a composition according to claims 1 to 8 for bleaching and colouring hair, especially human hair.

10. Use of an oxidizable solvent selected from 2-phenoxyethanol and benzyl alcohol for stabilizing the direct dye of the chemical structure 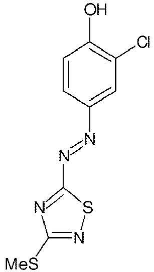 in an aqueous composition comprising at least one compound with hair bleaching effect, at least one oxidizing agent different from the compound with hair bleaching effect and at least one alkalizing agent.

11. Method for bleaching and colouring hair wherein an aqueous composition is prepared by mixing immediately before use wherein an anhydrous composition (Part A) comprising the dyestuff of the chemical structure 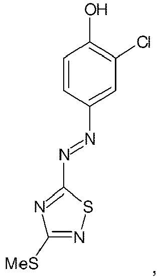 at least one compound with hair bleaching effect and at least one alkalizing agent, and an aqueous composition (Part B) comprising at least one oxidizing agent and a composition (Part C) comprising at least one oxidizable solvent and is applied onto hair and after processing 5 to 45 min rinsed off from the hair, wherein the oxidizable solvent is selected from 2-phenoxyethanol and benzyl alcohol.

12. Kit for coloring and bleaching hair comprising three of more compositions wherein it comprises a Part A which is an anhydrous composition and comprises the dyestuff of the chemical structure 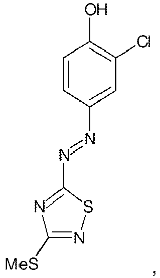 at least one compound with bleaching effect and at least one alkalizing agent, and a Part B which is an aqueous composition comprising at least one oxidizing agent and a Part C comprising at least one oxidizable solvent selected from 2-phenoxyethanol and benzyl alcohol.

## Patentansprüche

1. Wässrige Zusammensetzung zum Bleichen und Färben von Haaren, **dadurch gekennzeichnet, dass** sie mindestens eine Verbindung mit haarbleichender Wirkung, mindestens ein Oxidationsmittel, das von der Verbindung mit haarbleichender Wirkung verschieden ist, mindestens ein Alkalisierungsmittel und mindestens ein oxidierbares Lösungsmittel, ausgewählt aus 2-Phenoxyethanol und Benzylalkohol, und den Farbstoff der chemischen Struktur 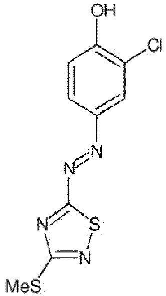 umfasst.

2. Zusammensetzung gemäß Anspruch 1, **dadurch gekennzeichnet, dass** mindestens eine Verbindung mit bleichender Wirkung aus Persulfaten, wie beispielsweise Natriumpersulfat, Kaliumpersulfat und Ammoniumpersulfat oder deren Mischungen, ausgewählt ist und vorzugsweise in einer Konzentration von mindestens 5 Gew.-%, berechnet auf die Gesamtzusammensetzung, vorliegt.

3. Zusammensetzung gemäß einem der Ansprüche 1 und 2, **dadurch gekennzeichnet, dass** sie zusätzlich mindestens einen Direktfarbstoff, ausgewählt aus kationischen, anionischen und nichtionischen Farbstoffen, umfasst.

4. Zusammensetzung gemäß einem der vorhergehenden Ansprüche, **dadurch gekennzeichnet, dass** mindestens ein zusätzlicher Direktfarbstoff ein anionischer Direktfarbstoff ist und vorzugsweise aus Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 und Disperse Violet 1 sowie deren Alkalimetallsalzen wie Natrium und Kalium ausgewählt ist. Von diesen sind die am meisten bevorzugten anionischen Farbstoffe Acid Red 52, DC Violet 2, DC Red 33, DC Orange 4, DC Red 27, DC Yellow 10,

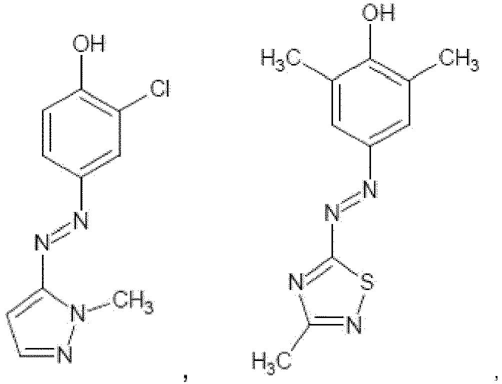

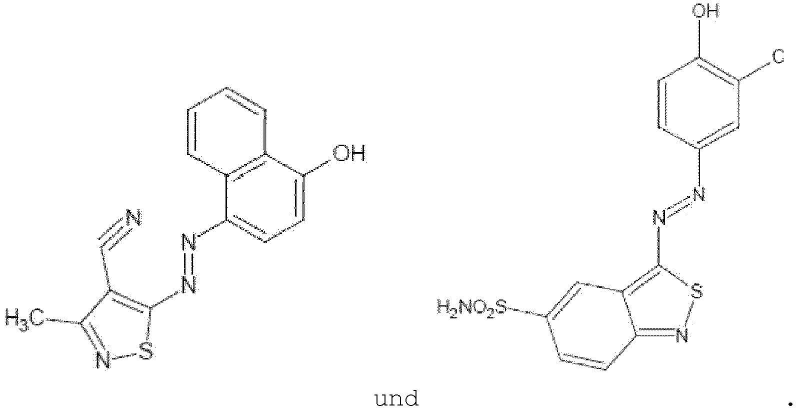

5. Zusammensetzung gemäß einem der vorhergehenden Ansprüche, **dadurch gekennzeichnet, dass** mindestens ein Oxidationsmittel Wasserstoffperoxid ist.

6. Zusammensetzung gemäß einem der vorangehenden Ansprüche, **dadurch gekennzeichnet, dass** mindestens ein Alkalisierungsmittel Natriummetasilicat ist.

7. Zusammensetzung gemäß einem der vorhergehenden Ansprüche, **dadurch gekennzeichnet, dass** mindestens ein oxidierbares Lösungsmittel in einer Konzentration von weniger als 8 Gew.-%, bevorzugt 0,1 bis 7,5 Gew.-% und mehr bevorzugt 0,2 bis 6 Gew.-% und am meisten bevorzugt 0,25 bis 5 Gew.-%, bezogen auf die gesamte Zusammensetzung, enthalten ist.

8. Zusammensetzung gemäß einem der vorangehenden Ansprüche, **dadurch gekennzeichnet, dass** sie mindestens ein kationisches Polymer umfasst.

9. Verwendung einer Zusammensetzung gemäß einem der Ansprüche 1 bis 8 zum Bleichen und Färben von Haaren, insbesondere menschlichen Haaren.

10. Verwendung eines oxidierbaren Lösungsmittels, ausgewählt aus 2-Phenoxyethanol und Benzylalkohol, zur Stabilisierung des Direktfarbstoffs der chemischen Struktur 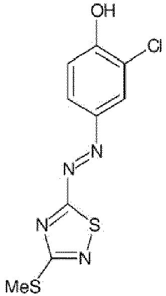 in einer wässrigen Zusammensetzung, umfassend mindestens eine Verbindung mit haarbleichender Wirkung, mindestens ein Oxidationsmittel, das von der Verbindung mit haarbleichender Wirkung verschieden ist, und mindestens ein Alkalisierungsmittel.

11. Verfahren zum Bleichen und Färben von Haaren, bei dem eine wässrige Zusammensetzung durch Mischen unmittelbar vor der Anwendung hergestellt, wobei eine wasserfreie Zusammensetzung (Teil A) den Farbstoff der chemischen Struktur 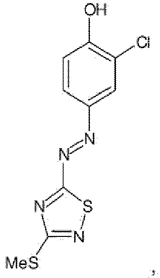 mindestens eine Verbindung mit haarbleichender Wirkung und mindestens ein Alkalisierungsmittel umfasst, und eine wässrige Zusammensetzung (Teil B) mindestens ein Oxidationsmittel umfasst, und eine Zusammensetzung (Teil C) mindestens ein oxidierbares Lösungsmittel umfasst, und auf das Haar aufgetragen und nach dem Wirken für 5 bis 45 Minuten vom Haar abgespült wird, wobei das oxidierbare Lösungsmittel aus 2-Phenoxyethanol und Benzylalkohol ausgewählt ist.

12. Kit zum Färben und Bleichen von Haaren, umfassend drei oder mehr Zusammensetzungen, wobei es einen Teil A, der eine wasserfreie Zusammensetzung ist und den Farbstoff mit der folgenden chemischen Struktur 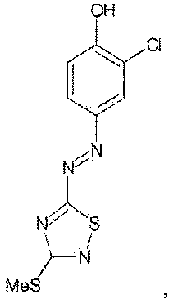 mindestens eine Verbindung mit bleichender Wirkung und mindestens ein Alkalisierungsmittel umfasst, und einen Teil B, der eine wässrige Zusammensetzung ist und mindestens ein Oxidationsmittel umfasst, und einen Teil C, der mindestens ein oxidierbares Lösungsmittel, ausgewählt aus 2-Phenoxyethanol und Benzylalkohol, umfasst, umfasst.

## Revendications

1. Composition aqueuse pour décolorer et colorer les cheveux, **caractérisée en ce qu'**elle comprend au moins un composé avec un effet de décoloration des cheveux, au moins un agent oxydant différent du composé avec l'effet de décoloration des cheveux, au moins un agent alcalifiant et au moins un solvant oxydable sélectionné parmi le 2-phénoxyéthanol et l'alcool benzylique et la matière colorante de la structure chimique

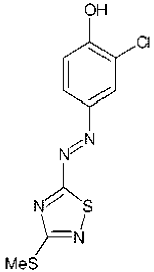

2. Composition selon la revendication 1, **caractérisée en ce qu'**au moins un composé avec un effet de décoloration est sélectionné parmi des persulfates tels que le persulfate de sodium, le persulfate de potassium et le persulfate d'ammonium ou leurs mélanges, et de préférence en une concentration d'au moins 5 % en poids calculée par rapport à la composition totale.

3. Composition selon les revendications 1 et 2, **caractérisée en ce qu'**elle comprend additionnellement au moins un colorant direct sélectionné parmi des colorants cationiques, anioniques et non ioniques.

4. Composition selon l'une quelconque des revendications précédentes, **caractérisée en ce qu'**au moins un colorant direct supplémentaire est un colorant direct anionique et de préférence sélectionné parmi Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 et Disperse Violet 1 et leurs sels de métaux alcalins tels que le sodium et le potassium. Parmi ceux-ci, les matières colorantes anioniques préférées entre toutes sont Acid Red 52, DC Violet 2, DC Red 33, DC Orange 4, DC Red 27, DC Yellow 10.

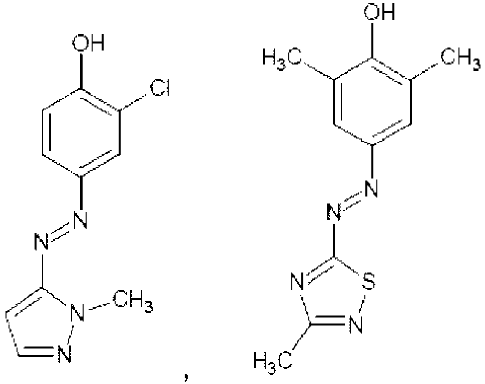

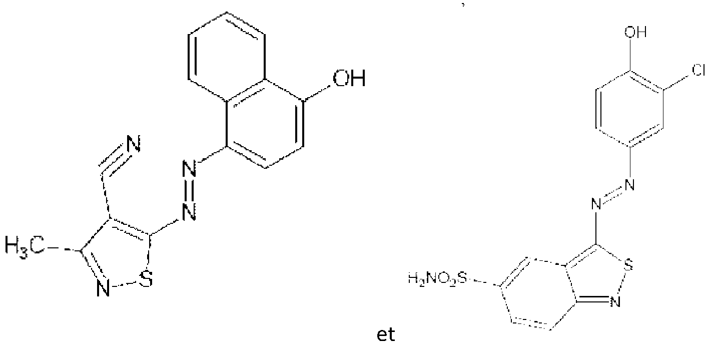

5. Composition selon l'une quelconque des revendications précédentes, **caractérisée en ce qu'**au moins un agent oxydant est le peroxyde d'hydrogène.

6. Composition selon l'une quelconque des revendications précédentes, **caractérisée en ce qu'**au moins un agent alcalifiant est le métasilicate de sodium.

7. Composition selon l'une quelconque des revendications précédentes, **caractérisée en ce qu'**au moins un solvant oxydable est compris en une concentration inférieure à 8 %, de préférence 0,1 à 7,5%, et plus préférentiellement 0,2 à 6 % et le plus préférentiellement 0,25 à 5 % en poids calculée par rapport à la composition totale.

8. Composition selon l'une quelconque des revendications précédentes, **caractérisée en ce qu'**elle comprend au moins un polymère cationique.

9. Utilisation d'une composition selon les revendications 1 à 8 pour décolorer et colorer les cheveux, en particulier les cheveux humains.

10. Utilisation d'un solvant oxydable sélectionné parmi le 2-phénoxyéthanol et l'alcool benzylique pour stabiliser le colorant direct de la structure chimique 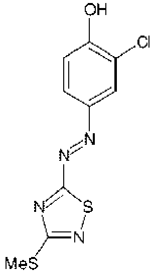 dans une composition aqueuse comprenant au moins un composé avec un effet de décoloration des cheveux, au moins un agent oxydant différent du composé avec un effet de décoloration des cheveux et au moins un agent alcalifiant.

11. Procédé de décoloration et de coloration des cheveux, dans lequel une composition aqueuse est préparée par mélange immédiatement avant utilisation, dans lequel une composition anhydre (Partie A) comprenant la matière colorante de la structure chimique 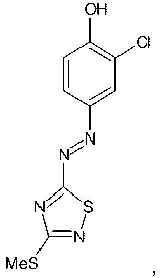 au moins un composé avec un effet de décoloration des cheveux et au moins un agent alcalifiant, et une composition aqueuse (Partie B) comprenant au moins un agent oxydant et une composition (Partie C) comprenant au moins un solvant oxydable et est appliquée sur les cheveux et après développement 5 à 45 minutes éliminée des cheveux par rinçage, dans lequel le solvant oxydable est sélectionné parmi le 2-phénoxyéthanol et l'alcool benzylique.

12. Kit de coloration et de décoloration des cheveux comprenant au moins trois compositions, dans lequel il comprend une Partie A qui est une composition anhydre et comprend la matière colorante de la structure chimique 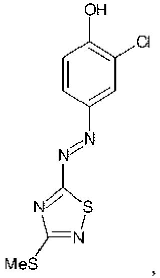 au moins un composé avec un effet de décoloration et au moins un agent alcalifiant, et une Partie B qui est une composition aqueuse comprenant au moins un agent oxydant et une Partie C comprenant au moins un solvant oxydable sélectionné parmi le 2-phénoxyéthanol et l'alcool benzylique.
